# EUROPÄISCHE PATENTSCHRIFT

(11) **EP 2 337 592 B1**
(45) Veröffentlichungstag und Bekanntmachung des Hinweises auf die Patenterteilung: **30.07.2014**
(21) Anmeldenummer: 09743850.1
(22) Anmeldetag: 29.09.2009
(51) Int. Cl.: A61M 1/30

(54) **VORRICHTUNG ZUR EXTRAKORPORALEN BLUTBEHANDLUNG IM EINNADEL-BETRIEB**
DEVICE FOR EXTRACORPORAL BLOOD TREATMENT IN SINGLE-NEEDLE OPERATING MODE
DISPOSITIF POUR LE TRAITEMENT EXTRACORPOREL DU SANG, EN FONCTIONNEMENT À UNE SEULE AIGUILLE

(30) Priorität: 02.10.2008 DE 102008050367
(43) Veröffentlichungstag der Anmeldung: 29.06.2011
(73) Patentinhaber: Fresenius Medical Care Deutschland GmbH, 61352 Bad Homburg v.d.H. (DE)
(72) Erfinder: KOPPERSCHMIDT, Pascal, 97456 Dittelbrunn (DE)
(74) Vertreter: Oppermann, Frank
(86) Internationale Anmeldenummer: PCT/EP2009/006994
(87) Internationale Veröffentlichungsnummer: WO 2010/037520

(56) Entgegenhaltungen:
- WO-A1-94/08687
- DE-C1- 4 039 084
- US-A- 4 904 234
- US-A1- 2004 199 098

## Beschreibung

Die Erfindung betrifft eine Vorrichtung zur extrakorporalen Blutbehandlung im Einnadel-Betrieb, bei der in einer arteriellen Phase Blut einem Patienten entnommen und in einer venösen Phase das Blut dem Patienten wieder zugeführt wird.

Extrakorporale Blutbehandlungsvorrichtungen mit einer Blutbehandlungseinheit, die von dem Blut eines Patienten durchströmt wird, sind allgemein bekannt. Zu diesen zählen beispielsweise die bekannten Hämodialyse-, Hämofiltrations- oder Hämodiafiltrationsvorrichtungen. Die bekannten Blutbehandlungsvorrichtungen können im Einnadel- oder Zweinadel-Betrieb betrieben werden.

Bei dem Einnadel-Betrieb wird das Blut mit einer einzigen Nadel dem Patienten entnommen, in die Blutbehandlungseinheit der Blutbehandlungsvorrichtung geleitet und über die einzige Nadel dem Patienten wieder zugeführt. Das dem Patienten entnommene Blut wird während einer arteriellen Phase in einem Reservoir gespeichert und in einer venösen Phase dem Patienten aus dem Reservoir wieder zugeführt. Dabei durchströmt das Blut die Blutbehandlungseinheit der Blutbehandlungsvorrichtung.

Für die Entnahme und Rückführung des Blutes finden auswechselbare Blutschlauchsysteme mit einer Blutzuführ- und -rückführleitung Verwendung, an denen die einzige Nadel (Kanüle) angeschlossen ist. Die auswechselbaren Schlauchsysteme werden in die Blutbehandlungsvorrichtung eingelegt und nach der Blutbehandlung verworfen. Daher werden diese Schlauchsysteme auch als Disposable bezeichnet. Auch die Blutbehandlungseinheit ist im Allgemeinen zur einmaligen Verwendung bestimmt.

Es sind Blutbehandlungsvorrichtungen für den Einnadel-Betrieb bekannt, die über nur eine einzige Blutpumpe verfügen, die in der arteriellen Blutzuführleitung angeordnet ist. Zum Sammeln des Bluts befindet sich ein Blutreservoir stromauf oder stromab der Blutbehandlungseinheit, das eine ausreichende Compliance hat. Neben den Blutbehandlungsvorrichtungen für den Einnadel-Betrieb mit nur einer einzigen Blutpumpe sind auch Blutbehandlungsvorrichtungen bekannt, die über zwei Blutpumpen verfügen, von denen die eine in der arteriellen Blutzuführleitung stromauf des Blutreservoirs und die andere Pumpe in der zu der Blutbehandlungseinheit führenden Blutzufiihrleitung stromab des Blutreservoirs angeordnet ist. Diese beiden Pumpen werden in der arteriellen und venösen Phase alternierend betrieben. In der arteriellen Phase wird das dem Patienten entnommene Blut mit der ersten Blutpumpe in das Blutreservoir gefördert, während in der venösen Phase das in dem Blutreservoir gesammelte Blut mit der zweiten Blutpumpe zu dem Patienten gefördert wird. Dabei durchströmt das Blut den Dialysator. Entscheidend ist, dass beim Betrieb der ersten Blutpumpe die zweite Blutpumpe stillsteht und bei dem Betrieb der zweiten Pumpe die erste Pumpe stillsteht. Dadurch wird die Blutzuführleitung stromauf oder stromab des Blutreservoirs jeweils unterbrochen. Eine Kompliance des Blutreservoirs ist beim Betrieb mit zwei Pumpen nicht erforderlich.

Blutbehandlungsvorrichtungen für den Einnadel-Betrieb mit einer oder zwei Blutpumpen sind beispielsweise in dem Fachbuch "Replacement Of Renal Function By Dialysis", 5th Revised Edition, ISBN 1-4020-0083-9, Kluwer Academic Publishers S. 365-367 beschrieben.

Aus der DE 196 33 657 C1 ist eine Dialysevorrichtung für den Einnadel-Betrieb bekannt, die über eine in der arteriellen Blutleitung angeordnete Blutpumpe verfügt, wobei das Blutreservoir einen in der arteriellen und einen in der venösen Blutleitung angeordneten Blutbeutel umfasst. Beide Blutbeutel werden mit einer Vorrichtung mit Druck beaufschlagt, so dass Blut aus den Blutbeuteln gefördert werden kann.

Kleinkinder und Säuglinge, die unter Nierenversagen leiden, werden aufgrund ihres geringen Körpervolumens mit nur relativ kleinen Blutflüssen behandelt. Dialysebehandlungen mit kontinuierlichen Blutflüssen, die relativ klein sind, beispielsweise bei etwa 5 ml/min liegen, sind mit Blutbehandlungsvorrichtungen, die über konventionelle Blutpumpen verfügen, im Allgemeinen nicht möglich, da die interne Regelung zur Stabilisierung der Motorumdrehung eine definierte Mindest-Umlaufgeschwindigkeit für den Pumpenrotor voraussetzt, um einen ruckfreien Betrieb zu gewährleisten. Daraus ergibt sich ein minimaler Blutfluss von etwa 20 ml/min. Dieser Blutfluss ist jedoch für Patienten mit einem geringen Körpergewicht, beispielsweise einem Körpergewicht von 10 kg zu groß.

Aus der US 2004/199098 A1 ist eine Vorrichtung zur Separation von Vollblut in Blut-komponenten bekannt. Der bekannte Zellseparator, der im Einnadel-Betrieb betrieben wird, weist eine arterielle Blutleitung auf, über die Vollblut, das dem Patienten mit einer Kanüle entnommen wird, einer Blutzentrifuge zugeführt wird, und eine venöse Blutleitung auf, über die ein Teil des Bluts wieder zu der Kanüle strömt. In der arteriellen und venösen Blutleitung befinden sich jeweils ein Blutsammelbeutel. Stromauf und stromab des Blutsammelbeutels befinden sich in der arteriellen Blutleitung eine Blutpumpe und in der venösen Blutleitung eine Blutpumpe. Die Blutpumpen in der venösen Blutleitung werden derart betrieben, das sich der Blutsammelbeutel während der arteriellen Phase mit Blut füllt. In der arteriellen Phase ist die Blutpumpe in der venösen Leitung nicht in Betrieb. Zum Rückführen der Blutkomponenten wird in der venösen Phase die Blutpumpe in der venösen Blutleitung in Betrieb gesetzt, während die Pumpe P1 in der arteriellen Leitung still steht. Folglich sieht der bekannnte Zellseparator einen alternierenden Betrieb in aufeinanderfolgenden Phasen vor, bei dem entweder Blut von der Kanüle in einen Blutssammelbeutel strömt oder Blut von einem Blutsammelbeutel zu der Nadel strömt.

Die US 4,904,234 beschreibt einen Plasmaseparator im Einnadel-Betrieb. Der Plasmaseparator verfügt über einen Rezirkulationskreislauf, der ein Blutsammelbehältnis und einen Separator einschließt. Das Blut wird mit einer Blutpumpe in den Rezirkulationskreislauf gefördert, die entweder zum Zuführen von Blut in den Rezirkulationskreislauf oder zum Rückführen von Blut aus dem Rezirkulationskreislauf dient. Insofern sieht die US 4,904,234 auch einen alternierenden Betrieb vor.

Einen sich wiederholenden zweistufigen Betrieb einer Einnadel-Dialysevorrichtung sieht auch die DE 40 39 084 C1 vor. Während der arteriellen Phase, in der dem Patienten Blut entnommen wird, ist die venöse Blutleitung mit einer Absperrklemme unterbrochen. Erst in der venösen Phase gibt die Absperrklemme den Rückfluss zum Patienten frei. Auch die WO 94/08687 sieht wieder einen alternierenden Betrieb einer Blutpumpe in der arteriellen Leitung und einer Pumpe in der venösen Leitung vor, so dass dem Patienten in alternierenden Phasen wechselweise Blut entnommen bzw. zugeführt wird.

Der Erfindung liegt die Aufgabe zugrunde, eine Vorrichtung zur extrakorporalen Blutbehandlung im Einnadel-Betrieb zu schaffen, die eine Blutbehandlung auch mit relativ kleinen Blutflüssen erlaubt.

Die Lösung dieser Aufgabe erfolgt erfindungsgemäß mit den Merkmalen des Patentanspruchs 1. Vorteilhafte Ausführungsformen der Erfindung sind Gegenstand der Unteransprüche.

Die erfindungsgemäße Vorrichtung zur extrakorporalen Blutbehandlung im Einnadel-Betrieb zeichnet sich dadurch aus, dass während der arteriellen und venösen Phase Blut sowohl in die Mittel zum Sammeln von Blut gefördert als auch Blut aus den Mitteln zum Sammeln von Blut gefördert wird. Die erfindungsgemäße Vorrichtung sieht also nicht einen alternierenden Betrieb der Mittel zum Fördern von Blut, sondern den gleichzeitigen Betrieb der Mittel zum Fördern von Blut in der arteriellen und venösen Phase vor.

Der gleichzeitige Betrieb beider Mittel zum Fördern von Blut erlaubt die Vorgabe von unterschiedlichen Förderraten. In der arteriellen Phase wird die Förderrate der Mittel zum Fördern von Blut in die Mittel zum Sammeln von Blut derart eingestellt, dass diese Förderrate größer als die Förderrate der Mittel zum Fördern von Blut aus den Mitteln zum Sammeln von Blut ist. Somit wird dem Patienten in der arteriellen Phase Blut entnommen.

In der venösen Phase hingegen ist die Förderrate der Mittel zum Fördern von Blut in die Mittel zum Sammeln von Blut kleiner als die Förderrate der Mittel zum Fördern von Blut aus den Mitteln zum Sammeln von Blut. Somit wird dem Patienten in der venösen Phase Blut zugeführt.

Die effektive Förderrrate ergibt sich aus der Differenz der beiden Förderarten. Da es nur auf die Differenz zwischen den Förderraten ankommt, können die Mittel zum Fördern von Blut mit relativ großen Förderraten betrieben werden, auch wenn die effektive Förderrate relativ klein ist. Daher ist der Einsatz von Blutpumpen, insbesondere peristaltischen Blutpumpen, beispielsweise Rollenpumpen möglich, die nur mit einer relativ großen Förderrate betrieben werden können, um eine Blutbehandlung mit einer relativ kleinen Blutflussrate durchführen zu können. Folglich lassen sich auch konventionelle Blutpumpen, deren minimale Förderrate über dem gewünschten Blutfluss liegt, verwenden.

Ein weiterer Vorteil besteht darin, dass relativ kleine Dialysatoren oder Filter verwendet werden können, da im extrakorporalen Blutkreislauf ein hoher Fluss herrscht und das Blut mehrfach durch den Dialysator oder Filter gepumpt wird (Rezirkulation). Der Fluss vom Patienten und zum Patienten ist dabei aber trotzdem klein.

Die Mittel zum Fördern von Blut können unterschiedlich ausgebildet sein. Vorzugsweise sind die Mittel zum Fördern von Blut Blutpumpen. Zum Fördern von Blut können aber auch Blutbeutel eingesetzt werden, die mit Druck beaufschlagt werden. Eine derartige Anordnung ist beispielsweise aus der DE 196 33 657 C1 bekannt.

Der Betrag der Differenz zwischen der Förderrate der Mittel zum Fördern von Blut in die Mittel zum Sammeln von Blut und der Förderrate der Mittel zum Fördern von Blut aus den Mitteln zum Sammeln von Blut sind vorzugsweise in der arteriellen Phase und in der venösen Phase gleich. Es ist aber auch möglich, dass der Betrag der Differenz zwischen der Förderrate der Mittel zum Fördern von Blut in die Mittel zum Sammeln von Blut und der Förderrate der Mittel zum Fördern von Blut aus den Mitteln zum Sammeln von Blut in der arteriellen Phase und in der venösen Phase unterschiedlich sind.

Die Mittel zum Sammeln von Blut können unterschiedlich ausgebildet sein. Beispielsweise können die Mittel zum Sammeln von Blut ein Blutbehälter, insbesondere ein Blutbeutel sein. Eine ausreichende Compliance ist aber nicht erforderlich, da das Blut mit zwei Pumpen gefördert wird.

Bei einer besonderen Ausführungsform der extrakorporalen Blutbehandlungsvorrichtung kann dem Blut ein Gemisch aus Plasma und löslichen Bestandteilen entzogen werden. Hierzu wird durch geeignete Mittel ein Druckgradient zwischen Blutkammer und Dialysierflüssigkeitskammer der Blutbehandlungseinheit aufgebaut, wodurch es zum Stoffübertritt aus dem Blut über die semipermeable Membran in das Dialysat kommt. Diesen Vorgang nennt man Ultrafiltration (Quelle: Die Dialysefibel 3 S786, ISBN 978-3-939508-88-5). Der Flüssigkeitsverlust des Blutes kann durch Zugabe einer Substituatslösung ganz, teilweise oder überkompensiert werden. Diesen Vorgang nennt man Postdilution, wenn die Zugabe der Substituatslösung stromabwärts der Blutbehandlungseinheit erfolgt, oder Prädilution, wenn die Zugabe der Substituatslösung stromaufwärts der Blutbehandlungseinheit erfolgt.

Eine bevorzugte Ausführungsform der extrakorporalen Blutbehandlungsvorrichtung verfügt über eine Eingabeeinheit zum Eingeben eines effektiven Blutflusses, der relativ klein sein kann, um eine Blutbehandlung bei Kleinkindern oder Säuglingen durchführen zu können. Die Steuereinheit der Blutbehandlungsvorrichtung ist derart ausgebildet, dass die Förderrate der Mittel zum Fördern von Blut in die Mittel zum Sammeln von Blut und die Förderrate der Mittel zum Fördern von Blut aus den Mitteln zum Sammeln von Blut derart eingestellt werden, dass in der venösen Phase die Differenz zwischen der Förderrate der Mittel zum Fördern von Blut aus den Mitteln zum Sammeln von Blut und der Förderrate der Mittel zum Fördern von Blut in die Mittel zum Sammeln von Blut gleich dem vorgegebenen effektiven Blutfluss sind.

Eine alternative bevorzugte Ausführungsform der extrakorporalen Blutbehandlungsvorrichtung verfügt über eine Eingabeeinheit zum Eingeben des effektiven Blutflusses, der relativ klein sein kann, in der arteriellen Phase und zum Eingeben eines anderen effektiven Blutflusses in der venösen Phase, der relativ klein sein kann und sich vom effektiven Blutfluss in der arteriellen Phase unterscheidet. Die Steuereinheit der Blutbehandlungsvorrichtung ist derart ausgebildet, dass die Förderrate der Mittel zum Förden von Blut in die Mittel zum Sammeln von Blut und die Förderrate der Mittel zum Förden von Blut aus den Mitteln zum Sammeln von Blut derart eingestellt werden, dass in der arteriellen Phase die Differenz zwischen der Förderrate der Mittel zum Förden von Blut in die Mittel zum Sammeln von Blut und der Förderrate der Mittel zum Fördern von Blut aus den Mitteln zum Sammeln von Blut gleich dem vorgegebenen effektiven Blutfluss der arteriellen Phase ist, und in der venösen Phase gleich dem vorgegebenen effektiven Blutfluss der venösen Phase ist.

Die Blutbehandlungseinheit und das Blutleitungssystem, das eine zu der Blutbehandlungseinheit führende arterielle Blutzuführleitung und eine von der Blutbehandlungseinheit abgehende venöse Blutrückführleitung umfasst, sind vorzugsweise nicht fester Bestandteil der extrakorporalen Blutbehandlungsvorrichtung, sondern sind zur einmaligen Verwendung bestimmt.

Bei einer weiteren bevorzugten Ausführungsform sind die Mittel zum Speichern von Blut in der arteriellen Blutzuführleitung angeordnet, die zu der Blutbehandlungseinheit der extrakorporalen Blutbehandlungsvorrichtung, insbesondere den Dialysator führt. Es ist aber grundsätzlich auch möglich, die Mittel zum Speichern von Blut in der venösen Blutleitung anzuordnen, die von der Blutbehandlungseinheit, insbesondere dem Dialysator abgeht. Allein entscheidend ist, dass die Mittel zum Sammeln von Blut zwischen den Mitteln zum Fördern von Blut in die Mittel zum Sammeln von Blut und den Mitteln zum Fördern von Blut aus den Mitteln zum Sammeln von Blut angeordnet sind, wobei die relative Lage der Blutbehandlungseinheit nicht relevant ist.

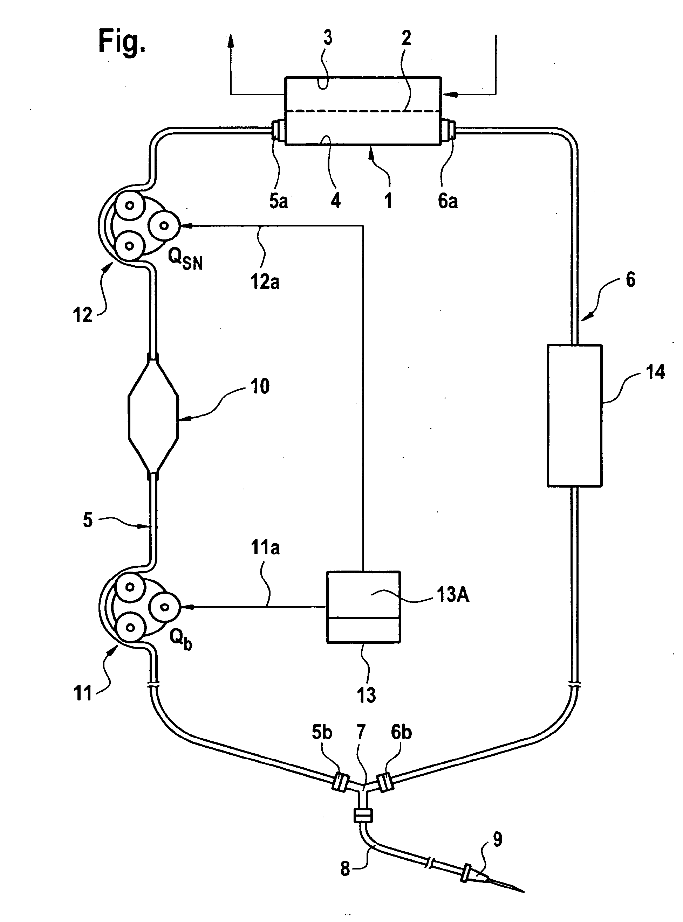

Im Folgenden wird ein Ausführungsbeispiel der Erfindung unter Bezugnahme auf die einzige Figur näher erläutert.
Die einzige Figur zeigt in stark vereinfachter schematischer Darstellung eine extrakorporale Blutbehandlungsvorrichtung für den Einnadel-Betrieb. In der Figur sind der besseren Übersichtlichkeit halber nur die für die Erfindung wesentlichen Komponenten der Blutbehandlungsvorrichtung dargestellt.

Die Blutbehandlungsvorrichtung, bei der es sich um eine Hämodialysevorrichtung handeln kann, weist als Blutbehandlungseinheit einen Dialysator 1 auf, der durch eine semipermeable Membran 2 in eine Dialysierflüssigkeitskammer 3 und eine Blutkammer 4 unterteilt ist. In der Figur ist nur der extrakorporale Blutkreislauf I dargestellt, der Dialysierflüssigkeitskreislauf der Hämodialysevorrichtung ist nur durch einen Pfeil angedeutet.

In die Blutbehandlungsvorrichtung wird ein Disposable eingelegt, das eine arterielle Blutzuführleitung 5 mit zwei Schlauchanschlüssen 5a und 5b, eine Blutrückführleitung 6 mit zwei Schlauchanschlüssen 6a und 6b, ein T-Verbindungsstück 7, eine gemeinsame Schlauchleitung 8 und eine Nadel (Hohlkanüle) 9 aufweist. Die Blutzuführleitung 5 ist einerseits an dem Einlass 5a der Blutkammer 4 des Dialysators 1 und andererseits an dem einen Verbindungszweig des T-Verbindungsstücks 7 angeschlossen, während die Blutrückführleitung 6 einerseits an dem Auslass 6a der Blutkammer 4 des Dialysators 1 und an dem anderen Verbindungszweig des T-Verbindungsstücks 7 angeschlossen ist. Der gemeinsame Verbindungszweig des T-Verbindungsstücks 7 ist über die gemeinsame Blutleitung 8 mit der Nadel 9 verbunden, die in ein Blutgefäß des Patienten eingestochen wird.

In die arterielle Blutzuführleitung 5 sind Mittel 10 zum Sammeln von Blut geschaltet, die als starrer Behälter ausgebildet sind. Der Blutsammelbehälter 10 ist Bestandteil des Blutschlauchsystems. In der venösen Blutrückführleitung 6 befindet sich ein Blasenfänger 14 (tropfkammer).

Das Blut des Patienten wird mit ersten Mitteln 11 in den Blutsammelbehälter 10 gefördert, während aus dem Blutsammelbehälter 10 das Blut mit zweiten Mitteln 12 gefördert wird. Die beiden Mittel 11 und 12 zum Fördern von Blut sind peristaltische Blutpumpen, insbesondere Rollenpumpen, in denen die arterielle Blutleitung 5 eingelegt ist. Beide Blutpumpen 11 und 12 sind über Steuerleitungen 11a, 12a mit einer zentralen Steuereinheit 13 der Blutbehandlungsvorrichtung verbunden, die für die Blutpumpe 11 stromauf des Blutsammelbehälters 10 und die Blutpumpe 12 stromab des Blutsammelbehälters 10 jeweils eine bestimmte Förderrate Q_{b}, Q_{SN} vorgibt.

Die zentrale Steuereinheit 13 der Blutbehandlungsvorrichtung ist derart ausgebildet, dass der Betrieb der Blutpumpen 11 und 12 während der extrakorporalen Blutbehandlung fortlaufend zwischen einer arteriellen und einer venösen Phase umgeschaltet wird. In der arteriellen Phase stellt die Steuereinheit 13 eine Förderrate Q_{ba} für die Pumpe 11 stromauf des Sammelbehälters 10 und eine Förderrate Q_{SNa} für die Pumpe 12 stromab des Sammelbehälters 10 ein, wobei die Förderrate Q_{ba} größer als die Förderrate Q_{SNa} ist. In der venösen Phase, die sich unmittelbar an die arterielle Phase anschließt, gibt die Steuereinheit 13 für die Pumpe 11 stromauf des Behälters 10 eine Förderrate Q_{bv} und für die Pumpe 12 stromab des Behälters 10 eine Förderrate Q_{SNv} vor, wobei die Förderrate Q_{bv} kleiner als die Förderrate Q_{SNv} ist. Die Differenz zwischen der Förderrate Q_{ba} der Pumpe 11 stromauf des Behälters 10 und der Förderrate Q_{SNa} der Pumpe 12 stromab des Behälters 10 in der arteriellen Phase ist die effektive arterielle Blutflussrate Q_{effa}, mit der die Blutbehandlung in der arteriellen Phase durchgeführt werden soll. Die Differenz zwischen der Förderrate Q_{SNv} der Pumpe 12 stromab des Behälters 10 und der Förderrate Q_{bv} der Pumpe 11 stromauf des Behälters 10 in der venösen Phase ist die effektive venöse Blutflussrate Q_{effv,} mit der die Blutbehandlung in der venösen Phase durchgeführt werden soll. Bei einer bevorzugten Ausführungsform ist die Dauer der arteriellen und venösen Phase gleich, die Dauer der arteriellen und venösen Phase kann aber auch unterschiedlich sein.

Die Steuereinheit 13 verfügt über eine Eingabeeinheit 13A, mit der die effektiven Blutflussraten Q_{effa} der arteriellen Phase und der Q_{effv} der venösen Phase eingestellt werden können. Zusätzlich können in die Eingabeeinheit 13A Werte für den SN-Pumpenfluss Q_{SNa} und Q_{SNv} oder Werte für die Blutpumpenflüsse Q_{ba} und Q_{bv} und der Wert für das Blutvolumen, das in der arteriellen Phase aus dem Blutgefäß entnommen wird V_{SN}, eingegeben werden.

Je nach Ausrüstungsgrad der Blutbehandlungsvorrichtung können überdies Werte für die Ultrafiltration und die Dilution von Substituatslösung eingegeben werden. Die Steuereinheit 13 errechnet aus diesen Eingaben die Steuersignale für die Mittel zum Fördern von Blut und für die Mittel, die die Ultrafiltration und die Substituatsdilution bewirken.

Bei einer beispielhaften Ausführungsform der Blutbehandlungsvorrichtung ohne Ultrafiltration und Substituatdilution und den Eingaben in die Steuereinheit 13A Q_{ba} = 55 ml/min, Q_{bv} = 50 ml/min, Q_{effa} = 5 ml und Q_{effv} = -5 ml stellt die Steuereinheit 13 die Förderraten Q_{ba} und Q_{SNa} so ein, dass in der arteriellen Phase Q_{ba} = 55 ml/min und Q_{SNa} = 50 ml/min ist und somit die Differenz der Förderraten Q_{eff} = Q_{ba} - Q_{SNa} = 5 ml/min positiv ist, was einen Blutfluss von der Kanüle 9 aus dem Blutgefäß in den extrakorporalen Blutkreislauf bedeutet. Für die venöse Phase stellt die Steuereinheit 13A die Förderraten Q_{bv} = 50 ml/min und Q_{SNv} = 55 ml/min ein, so dass die Differenz der Förderraten Q_{eff} = Q_{ba} - Q_{SNa} = -5 ml/min negativ ist, was einen Blutfluss aus dem extrakorporalen Blutkreislauf über die Kanüle 9 in das Blutgefäß bedeutet.

Die Steuereinheit 13 steuert die Dauer der einzelnen Phasen so, dass sich die zuvor eingegebenen Werte für das Volumen V_{SN}, die Ultrafiltration und die Substituatsdilution einstellen.

Die Umschaltpunkte zwischen der arteriellen und venösen Phase können auf der Grundlage der Überwachung des Drucks in dem Blutsammelbehälter 10 ermittelt werden.

Von Vorteil ist, wenn eine der beiden Blutpumpen 11 und 12 mit einer konstanten Förderrate betrieben wird, während die Förderate der anderen Blutpumpe in der arteriellen und venösen Phase jeweils derart verändert wird, dass sich die gewünschten Blutflüsse einstellen. Die Veränderung des Blutflusses nur einer der beiden Pumpen erfordert einen geringeren Aufwand für die Steuerung der Pumpen als wenn die Förderraten beider Pumpen in den jeweiligen Phasen verändert werden.

## Patentansprüche

1. Vorrichtung zur extrakorporalen Blutbehandlung für den Einnadel-Betrieb mit
Mitteln (11) zum Fördern von Blut in Mittel (10) zum Sammeln von Blut,
Mitteln (12) zum Fördern von Blut aus Mitteln (10) zum Sammeln von Blut,
einer Steuereinheit (13) zum Einstellen der Förderrate Q_{b} der Mittel (11) zum Fördern von Blut in die Mittel (10) zum Sammeln von Blut und der Förderrate Q_{SN} der Mittel (12) zum Fördern von Blut aus den Mitteln (10) zum Sammeln von Blut,
**dadurch gekennzeichnet,**
**dass** die Steuereinheit (13) derart ausgebildet ist, dass der Betrieb der Mittel (11, 12) zum Fördern von Blut zwischen einer arteriellen und venösen Phase fortlaufend umgeschaltet wird, wobei in der arteriellen Phase bei gleichzeitigem Betrieb der Mittel (11) zum Fördern von Blut in die Mittel (10) zum Sammeln von Blut und der Mittel (12) zum Fördern von Blut aus den Mitteln (10) zum Sammeln von Blut die Förderrate Q_{b} der Mittel (11) zum Fördern von Blut in die Mittel (10) zum Sammeln von Blut größer als die Förderrate Q_{SN} der Mittel (12) zum Fördern von Blut aus den Mitteln (10) zum Sammeln von Blut ist, so dass dem Patienten in der arteriellen Phase Blut entnommen werden kann,
und in der venösen Phase bei gleichzeitigem Betrieb der Mittel (11) zum Fördern von Blut in die Mittel (10) zum Sammeln von Blut und der Mittel (12) zum Fördern von Blut aus den Mitteln (10) zum Sammeln von Blut die Förderrate Q_{b} der Mittel (11) zum Fördern von Blut in die Mittel (10) zum Sammeln von Blut kleiner als die Förderrate Q_{SN} der Mittel (12) zum Fördern von Blut aus den Mitteln (10) zum Sammeln von Blut ist, so dass dem Patienten in der venösen Phase Blut zugeführt werden kann.

2. Vorrichtung nach Anspruch 1, **dadurch gekennzeichnet, dass** die Steuereinheit (13) derart ausgebildet ist, dass der Betrag der Differenz zwischen der Förderrate Q_{b} der Mittel (11) zum Fördern von Blut in die Mittel (10) zum Sammeln von Blut und der Förderrate Q_{SN} der Mittel (12) zum Fördern von Blut aus den Mitteln (10) zum Sammeln von Blut in der arteriellen Phase und der venösen Phase gleich sind.

3. Vorrichtung nach Anspruch 1, **dadurch gekennzeichnet, dass** die Steuereinheit (13) derart ausgebildet ist, dass der Betrag der Differenz zwischen der Förderrate Q_{b} der Mittel (11) zum Fördern von Blut in die Mittel (10) zum Sammeln von Blut und der Förderrate Q_{SN} der Mittel (12) zum Fördern von Blut aus den Mitteln (10) zum Sammeln von Blut in der arteriellen Phase und der venösen Phase unterschiedlich sind.

4. Vorrichtung nach Anspruch 2, **dadurch gekennzeichnet, dass** die extrakorporale Blutbehandlungsvorrichtung eine Eingabeeinheit (13A) zum Eingeben eines effektiven Blutflusses Q_{eff} aufweist, mit dem die Blutbehandlung durchgeführt werden soll, und dass die Steuereinheit (13) derart ausgebildet ist, dass die Förderrate Q_{b} der Mittel (11) zum Fördern von Blut in die Mittel (10) zum Sammeln von Blut und die Förderrate Q_{SN} der Mittel (12) zum Fördern von Blut aus den Mitteln (10) zum Sammeln von Blut derart eingestellt werden, dass in der venösen Phase die Differenz zwischen der Förderrate Q_{b} der Mittel (12) zum Fördern von Blut aus den Mitteln (10) zum Sammeln von Blut und der Förderrate Q_{SN} der Mittel (11) zum Fördern von Blut in die Mittel (10) zum Sammeln von Blut gleich dem vorgegebenen effektiven Blutfluss ist.

5. Vorrichtung nach Anspruch 3, **dadurch gekennzeichnet, dass** die extrakorporale Blutbehandlungsvorrichtung eine Eingabeeinheit (13A) zum Eingeben eines effektiven arteriellen und eines effektiven venösen Blutflusses Q_{effa,} Q_{effv} aufweist, mit dem die Blutbehandlung durchgeführt werden soll, und dass die Steuereinheit (13) derart ausgebildet ist, dass die Förderrate Q_{b} der Mittel (11) zum Fördern von Blut in die Mittel (10) zum Sammeln von Blut und die Förderrate Q_{SN} der Mittel (12) zum Fördern von Blut aus den Mitteln (10) zum Sammeln von Blut derart eingestellt werden, dass in der arteriellen Phase die Differenz zwischen der Förderrate Q_{b} der Mittel (11) zum Fördern von Blut in die Mittel (10) zum Sammeln von Blut und der Förderrate Q_{SN} der Mittel (12) zum Fördern von Blut aus den Mitteln (10) zum Sammeln von Blut gleich dem vorgegebenen effektiven arteriellen Blutfluss Q_{effa} ist und in der venösen Phase die Differenz zwischen der Förderrate Q_{SN} der Mittel (12) zum Fördern von Blut aus den Mitteln (10) zum Sammeln von Blut und der Förderrate Q_{b} der Mittel (11) zum Fördern von Blut in die Mittel (10) zum Sammeln von Blut gleich dem vorgegebenen effektiven venösen Blutfluss Q_{effv} ist.

6. Vorrichtung nach einem der Ansprüche 1 bis 5, **dadurch gekennzeichnet, dass** die extrakorporale Blutbehandlungsvorrichtung eine Blutbehandlungseinheit (1) mit einer Blutkammer (4), die einen Einlass (5a) und einen Auslass (6a) hat, und ein Blutleitungsystem (5,6) aufweist, wobei das Blutleitungssystem eine arterielle Blutleitung (5), die von einer Nadel (9) zu dem Einlass (5a) der Blutkammer (4) der Blutbehandlungseinheit (1) führt, und eine venöse Blutleitung (6) aufweist, die von der Blutkammer (4) der Blutbehandlungseinheit (1) zu der Nadel führt.

7. Vorrichtung nach einem der Ansprüche 1 bis 6, **dadurch gekennzeichnet, dass** die Mittel (10) zum Speichern von Blut in der arteriellen Blutleitung (5) angeordnet sind.

8. Vorrichtung nach einem der Ansprüche 1 bis 7, **dadurch gekennzeichnet, dass** die Mittel zum Fördern von Blut in die Mittel (10) zum Sammeln von Blut eine Blutpumpe (11), insbesondere eine peristaltische Blutpumpe sind.

9. Vorrichtung nach einem der Ansprüche 1 bis 8, **dadurch gekennzeichnet, dass** die Mittel zum Fördern von Blut aus den Mitteln (10) zum Sammeln von Blut eine Blutpumpe (12), insbesondere eine peristaltische Blutpumpe sind.

## Claims

1. Device for extracorporeal blood treatment for single-needle operation, comprising means (11) for conveying blood into means (10) for collecting blood, means (12) for conveying blood out of means (10) for collecting blood, a control unit (13) for adjusting the delivery rate Q_{b} of the means (11) for conveying blood into the means (10) for collecting blood and for adjusting the delivery rate Q_{SN} of the means (12) for conveying blood out of the means (10) for collecting blood, **characterised in that** the control unit (13) is configured such that the operation of the means (11, 12) for conveying blood is continuously switched between an arterial phase and venous phase, in the arterial phase, when the means (11) for conveying blood into the means (10) for collecting blood and the means (12) for conveying blood out of the means (10) for collecting blood are operated simultaneously, the delivery rate Q_{b} of the means (11) for conveying blood into the means (10) for collecting blood being greater than the delivery rate Q_{SN} of the means (12) for conveying blood out of the means (10) for collecting blood, so that blood can be removed from the patient in the arterial phase, and, in the venous phase, when the means (11) for conveying blood into the means (10) for collecting blood and the means (12) for conveying blood out of the means (10) for collecting blood are operated simultaneously, the delivery rate Q_{b} of the means (11) for conveying blood into the means (10) for collecting blood being lower than the delivery rate Q_{SN} of the means (12) for conveying blood out of the means (10) for collecting blood, so that blood can be supplied to the patient in the venous phase.

2. Device according to claim 1, **characterised in that** the control unit (13) is configured such that the value of the difference between the delivery rate Q_{b} of the means (11) for conveying blood into the means (10) for collecting blood and the delivery rate Q_{SN} of the means (12) for conveying blood out of the means (10) for collecting blood is the same in the arterial phase and the venous phase.

3. Device according to claim 1, **characterised in that** the control unit (13) is configured such that the value of the difference between the delivery rate Q_{b} of the means (11) for conveying blood into the means (10) for collecting blood and the delivery rate Q_{SN} of the means (12) for conveying blood out of the means (10) for collecting blood is different in the arterial phase and the venous phase.

4. Device according to claim 2, **characterised in that** the extracorporeal blood treatment device comprises an input unit (13A) for inputting an effective blood flow Q_{eff} with which the blood treatment is to be carried out, and **in that** the control unit (13) is configured such that the delivery rate Q_{b} of the means (11) for conveying blood into the means (10) for collecting blood and the delivery rate Q_{SN} of the means (12) for conveying blood out of the means (10) for collecting blood are adjusted in such a way that, in the venous phase, the difference between the delivery rate Q_{b} of the means (12) for conveying blood out of the means (10) for collecting blood and the delivery rate Q_{SN} of the means (11) for conveying blood into the means (10) for collecting blood is equal to the predetermined effective blood flow.

5. Device according to claim 3, **characterised in that** the extracorporeal blood treatment device comprises an input unit (13A) for inputting an effective arterial and an effective venous blood flow Q_{effa}, Q_{effv} with which the blood treatment is to be carried out, and **in that** the control unit (13) is configured such that the delivery rate Q_{b} of the means (11) for conveying blood into the means (10) for collecting blood and the delivery rate Q_{SN} of the means (12) for conveying blood out of the means (10) for collecting blood are adjusted in such a way that, in the arterial phase, the difference between the delivery rate Q_{b} of the means (11) for conveying blood into the means (10) for collecting blood and the delivery rate Q_{SN} of the means (12) for conveying blood out of the means (10) for collecting blood is equal to the predetermined effective arterial blood flow Q_{effa} and, in the venous phase, the difference between the delivery rate Q_{SN} of the means (12) for conveying blood out of the means (10) for collecting blood and the delivery rate Q_{b} of the means (11) for conveying blood into the means (10) for collecting blood is equal to the predetermined effective venous blood flow Q_{effv}.

6. Device according to any of claims 1 to 5, **characterised in that** the extracorporeal blood treatment device has a blood treatment unit (1) having a blood chamber (4), which comprises an inlet (5a) and an outlet (6a), and a blood line system (5, 6), the blood line system comprising an arterial blood line (5), which leads from a needle (9) to the inlet (5a) of the blood chamber (4) of the blood treatment unit (1), and a venous blood line (6), which leads from the blood chamber (4) of the blood treatment unit (1) to the needle.

7. Device according to any of claims 1 to 6, **characterised in that** the means (10) for storing blood are arranged in the arterial blood line (5).

8. Device according to any of claims 1 to 7, **characterised in that** the means for conveying blood into the means (10) for collecting blood is a blood pump (11), more particularly a peristaltic blood pump.

9. Device according to any of claims 1 to 8, **characterised in that** the means for conveying blood out of the means (10) for collecting blood is a blood pump (12), more particularly a peristaltic blood pump.

## Revendications

1. Dispositif pour le traitement extracorporel du sang pour un fonctionnement à aiguille unique, comportant
des moyens (11) pour le refoulement du sang dans des moyens (10) pour la collecte du sang,
des moyens (12) pour le refoulement du sang à partir des moyens (10) pour la collecte du sang,
une unité de commande (13) pour l'ajustement du débit de refoulement Q_{b} des moyens (11) pour le refoulement du sang dans les moyens (10) pour la collecte du sang, et du débit de refoulement Q_{SN} des moyens (12) pour le refoulement du sang à partir des moyens (10) pour la collecte du sang,
**caractérisé en ce que**
l'unité de commande (13) est configurée de telle sorte que le fonctionnement des moyens (11, 12) pour le refoulement du sang subisse une commutation continue entre une phase artérielle et une phase veineuse, le débit de refoulement Q_{b} des moyens (11) pour le refoulement du sang dans les moyens (10) pour la collecte du sang étant, dans la phase artérielle, dans le cas d'un fonctionnement simultané des moyens (11) pour le refoulement du sang dans les moyens (10) pour la collecte du sang et des moyens (12) pour le refoulement du sang à partir des moyens (10) pour la collecte du sang, supérieur au débit de refoulement Q_{SN} des moyens (12) pour le refoulement du sang à partir des moyens (10) pour la collecte du sang, de telle sorte que du sang puisse être prélevé du patient dans la phase artérielle,
et, dans la phase veineuse, dans le cas d'un fonctionnement simultané des moyens (11) pour le refoulement du sang dans les moyens (10) pour la collecte du sang et des moyens (12) pour le refoulement du sang à partir des moyens (10) pour la collecte du sang, le débit de refoulement Q_{b} des moyens (11) pour le refoulement du sang dans les moyens (10) pour la collecte du sang étant inférieur au débit de refoulement Q_{SN} des moyens (12) pour le refoulement du sang à partir des moyens (10) pour la collecte du sang, de telle sorte que du sang puisse être amené au patient dans la phase veineuse.

2. Dispositif selon la revendication 1, **caractérisé en ce que** l'unité de commande (13) est configurée de telle sorte que la valeur de la différence entre le débit de refoulement Q_{b} des moyens (11) pour le refoulement du sang dans les moyens (10) pour la collecte du sang et le débit de refoulement Q_{SN} des moyens (12) pour le refoulement du sang à partir des moyens (10) pour la collecte du sang, soit la même dans la phase artérielle et dans la phase veineuse.

3. Dispositif selon la revendication 1, **caractérisé en ce que** l'unité de commande (13) est configurée de telle sorte que la valeur de la différence entre le débit de refoulement Q_{b} des moyens (11) pour le refoulement du sang dans les moyens (10) pour la collecte du sang et le débit de refoulement Q_{SN} des moyens (12) pour le refoulement du sang à partir des moyens (10) pour la collecte du sang, soit différente dans la phase artérielle et dans la phase veineuse.

4. Dispositif selon la revendication 2, **caractérisé en ce que** le dispositif de traitement extracorporel du sang comporte une unité d'entrée (13A) pour introduire un débit sanguin effectif Q_{eff}, à l'aide duquel doit être mis en oeuvre le traitement du sang, et que l'unité de commande (13) est configurée de telle sorte que le débit de refoulement Q_{b} des moyens (11) pour le refoulement du sang dans les moyens (10) pour la collecte du sang et le débit de refoulement Q_{SN} des moyens (12) pour le refoulement du sang à partir des moyens (10) pour la collecte du sang, soient ajustés de telle sorte que, dans la phase veineuse, la différence entre le débit de refoulement Q_{b} des moyens (12) pour le refoulement du sang à partir des moyens (10) pour la collecte du sang et le débit de refoulement Q_{SN} des moyens (11) pour le refoulement du sang dans les moyens (10) pour la collecte du sang, soit égale au débit sanguin effectif prédéfini.

5. Dispositif selon la revendication 3, **caractérisé en ce que** le dispositif de traitement extracorporel du sang comprend une unité d'entrée (13A) pour introduire un débit sanguin artériel effectif et un débit sanguin veineux effectif Q_{effa}, Q_{effv}, à l'aide duquel doit être mis en oeuvre le traitement du sang, et que l'unité de commande (13) est configurée de telle sorte que le débit de refoulement Q_{b} des moyens (11) pour le refoulement du sang dans les moyens (10) pour la collecte du sang et le débit de refoulement Q_{SN} des moyens (12) pour le refoulement du sang à partir des moyens (10) pour la collecte du sang, soient ajustés de telle sorte que, dans la phase artérielle, la différence entre le débit de refoulement Q_{b} des moyens (11) pour le refoulement du sang dans les moyens (10) pour la collecte du sang et le débit de refoulement Q_{SN} des moyens (12) pour le refoulement du sang à partir des moyens (10) pour la collecte du sang soit égale au débit sanguin artériel effectif Q_{effa} prédéfini, et que, dans la phase veineuse, la différence entre le débit de refoulement Q_{SN} des moyens (12) pour le refoulement du sang à partir des moyens (10) pour la collecte du sang et le débit de refoulement Q_{b} des moyens (11) pour le refoulement du sang dans les moyens (10) pour la collecte du sang soit égale au débit sanguin veineux effectif Q_{effv} prédéfini.

6. Dispositif selon l'une des revendications 1 à 5, **caractérisé en ce que** le dispositif de traitement extracorporel du sang comprend une unité de traitement du sang(1), comportant une chambre à sang (4), qui présente un orifice d'entrée (5a) et un orifice de sortie (6a), et un système de lignes de sang (5, 6), le système de lignes de sang comprenant une ligne de sang artérielle (5), qui va d'une aiguille (9) à l'orifice d'entrée (5a) de la chambre à sang (4) de l'unité (1) de traitement du sang, et une ligne de sang veineuse (6), qui va de la chambre à sang (4) de l'unité (1) de traitement du sang à l'aiguille.

7. Dispositif selon l'une des revendications 1 à 6, **caractérisé en ce que** les moyens (10) pour le stockage du sang sont disposés dans la ligne de sang artérielle (5).

8. Dispositif selon l'une des revendications 1 à 7, **caractérisé en ce que** les moyens pour le refoulement du sang dans les moyens (10) pour la collecte du sang sont une pompe sanguine (11), en particulier une pompe à sang péristaltique.

9. Dispositif selon l'une des revendications 1 à 8, **caractérisé en ce que** les moyens pour le refoulement du sang à partir des moyens (10) pour la collecte du sang sont une pompe sanguine (12), en particulier une pompe à sang péristaltique.
